# EUROPÄISCHE PATENTSCHRIFT

(11) **EP 0 735 992 B1**
(45) Veröffentlichungstag und Bekanntmachung des Hinweises auf die Patenterteilung: **03.01.2001**
(21) Anmeldenummer: 95937846.4
(22) Anmeldetag: 25.10.1995
(51) Int. Cl.: C07B 39/00, C07C 17/16, C07C 22/08, C07J 1/00, C07J 9/00

(54) **VERFAHREN ZUR UMWANDLUNG VON HYDROXYLGRUPPEN IN DIE ENTSPRECHENDEN FLUORVERBINDUNGEN**
METHOD OF CONVERTING HYDROXYL GROUPS INTO CORRESPONDING FLUORO COMPOUNDS
PROCEDE PERMETTANT DE CONVERTIR DES GROUPES HYDROXYLE EN COMPOSES FLUORES CORRESPONDANTS

(30) Priorität: 26.10.1994 DE 4439488
(43) Veröffentlichungstag der Anmeldung: 09.10.1996
(73) Patentinhaber: BAYER AG, 51368 Leverkusen (DE)
(72) Erfinder: VORBRÜGGEN, Helmut, D-13507 Berlin (DE); BENNUA-SKALMOWSKI, Bärbel, D-14167 Berlin (DE)
(86) Internationale Anmeldenummer: EP9504192
(87) Internationale Veröffentlichungsnummer: WO9613474

(56) Entgegenhaltungen:
- US-A- 3 914 265
- BULLETIN DES SOCI T S CHIMIQUES BELGES, Bd. 103, Nr. 7-8, Juli 1994 Seiten 453-461, BÄRBEL BENNUA-SKALMOWSKI ET AL 'The reaction of perfluorobutanesulfonyl fluoride with alcohols in the presence of 4-dialkylaminopyridines'
- TETRAHEDRON LETTERS, Bd. 36, Nr. 15, 10.April 1995 OXFORD GB, Seiten 2611-2614, B.BENNUA-SKALMOWSKI ET AL 'A facile conversion of primary and secondary alcohols with n-perfluorobutanesulfonyl fluoride/1,8-diazabicyclo[5.4.0]undec-7-en e into their corresponding fluorides' in der Anmeldung erwähnt

## Beschreibung

Die Erfindung betrifft neue Verfahren zur Umwandlung von Hydroxylgruppen in die entsprechenden Fluorverbindungen, wobei primäre und sekundäre Alkohole mit Perfluorbutansulfonylfluorid oder höheren Homologen, wie Perfluoroktansulfonylfluorid, in Gegenwart von 2-3 Äquivalenten starker organischer Basen in absoluten organischen Lösungsmitteln umgesetzt werden.

Bei den gegenwärtigen am häufigsten durchgeführten Verfahren zur Einführung von Fluoratomen in organische Moleküle werden primäre und sekundäre aliphatische Hydroxylgruppen mit Diäthylaminoschwefeltrifluorid (DAST) in einer Reaktionsstufe zu den entsprechenden Fluorverbindungen umgesetzt (M. Hudlicky, *Organic Reactions* **35**, 513; J. A. Wilkinson, *Chem. Rev*. **92**, 505 - 519 (1992)).

Der Nachteil dieser Verfahren besteht darin, daß das bei der Reaktion zur Anwendung kommende DAST relativ teuer ist. Ferner liegen die Ausbeuten an den entsprechenden Fluorverbindungen, wie zum Beispiel bei der Reaktion von 3-β-Hydroxycholestanol mit DAST zum 3-α-Fluor-cholestan, nur bei 40 %.

Aus "Bulletin des Sociétés Chimiques Belges", Bd. 103, Nr. 7-8, 1994, S. 453-461, ist bekannt, dass Alkohole durch Reaktion mit Perfluorbutanylsulfonylfluorid in die entsprechenden Fluoride überführt werden können.

Es wurde nun überraschend gefunden, daß bei Sulfonylierungsreaktionen mit dem großtechnisch hergestellten Perfluorbutansulfonylfluorid oder höheren Homologen, wie Perfluoroktansulfonylfluorid, primäre und sekundäre Alkohole in Gegenwart von 2 - 3 Äquivalenten starker organischer Basen, wie zum Beispiel 1,3-Diazabicyclo[5.4.0]undecen (DBU) oder 1,3-Diazabicyclo[4.3.0]nonen (DBN) sowie Pentaalkylguanidinen, in absoluten organischen Lösungsmitteln, wie zum Beispiel Toluol, glatt und in teils ausgezeichneten Ausbeuten zu den entsprechenden Fluorverbindungen umgesetzt werden können.

Gegenstand der vorliegenden Erfindung sind somit Verfahren zur Herstellung von Fluorverbindungen, die dadurch gekennzeichnet sind, daß man Hydroxy-Aliphaten der allgemeinen Formel **1** mit 1 - 2 Äquivalenten Perfluorbutansulfonylfluorid **2** oder höheren Homologen, wie Perfluoroktansulfonylfluorid, in Gegenwart von 2-3 Äquivalenten einer starken organischen (org.) Base **3** in einem indifferenten organischen Lösungsmittel zu den entsprechenden Fluorverbindungen der allgemeinen Formel **4**, in der
- R₁: für einen gegebenenfalls substituierten gradkettigen oder verzweigten, aliphatischen oder araliphatischen organischen Rest und
- R₂ und R₃: für Wasserstoff stehen,
oder
- R₁ und R₂: gegebenenfalls substituierte gradkettige oder verzweigte aliphatische oder araliphatische Reste darstellen und
- R₃: Wasserstoff ist,
bedeuten, gemäß der Reaktion 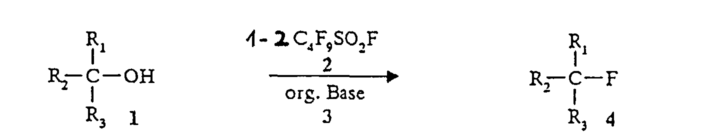 umsetzt.

Aliphatische oder araliphatische polycyclische Ringsysteme sind zum Beispiel 3-Hydroxy- oder 17-Hydroxy-Steroide oder Hydroxyprostanester.

Als starke organische Basen kommen prinzipiell alle Basen mit einem P_{Ka} ≥ 12 in Frage, wie z. B. alle Amidin- bzw. Guanidinbasen, wie DBU, DBN, Pentamethyl- oder Pentaisopropylguanidin, die *keine* reaktiven NH-Gruppen enthalten dürfen, und die neuen Phosphin-iminbasen (Schwesinger-Basen) wie das tert.-Butyliminotris(dimethylamino)phosphoran und das 1-tert.-Butyl-4,4,4-tris(dimethylamino)-2,2-bis-[tris(dimethylamino)phosphoranylideneamino]-2λ⁵, 4λ⁵-catenadi(phosphazen).

Die Reaktanden werden in einem indifferenten wasserfreien Lösungsmittel wie Toluol, Benzol, Xylol, Anisol, Diäthyläther, Tetrahydrofuran, 1,4-Dioxan, Methyltert.-butyläther, Acetonitril, Sulfolan oder Essigester vorzugsweise bei -10 ° → +24 °C umgesetzt, wobei das flüchtige n-Perfluorbutansulfonylfluorid **2** (Kp. 64 °C) langsam, unter Rühren zu der Lösung oder Suspension einer Hydroxyverbindung **1** und einer starken organischen Base **3** zugetropft wird. Überraschend ist die starke Abhängigkeit der Reaktionsgeschwindigkeit der Fluoridbildung von der Konfiguration des sekundären Alkohols. So setzt sich 3-β-Hydroxycholestan mit einer äquatorialen Hydroxygruppe sehr glatt in ca. 60 % Ausbeute zum 3-α-Fluorcholestan um, während das 3-α-Hydroxyandrostan mit einer axialen Hydroxylgruppe sehr viel langsamer zum 3-β-Fluorandrostan umgesetzt wird.

Die über das Verfahren herstellbaren Verbindungen sind u. a. von großem Interesse für die Herstellung von Pharmazeutica und Agrochemikalien (s. R.E. Banks, D.W.A. Sharp und l.C. Tatlow, Herausgeber: Fluorine: The First Hundred Years, Elsevier Seq., 1986) sowie von Fluorpharmaka (I.T. Welck, S. Esra-Kishman,Fluorine in Bioorganic Chemistry, John Wiley, N.Y., 1991).

Die nachfolgenden Ausführungsbeispiele erläutern das erfindungsgemäße Verfahren ohne es jedoch einzuschränken.

### BEISPIELE

### 1. Herstellung von 3-Fluorpropylbenzol.

Zu einer Lösung von 1,36 g (10 mmol) 3-Phenyl-1-propanol und 4.56 g (30 mmol) DBU in 80 ml abs. Toluol wurden 2,7 ml (15 mmol) Perfluorbutansulfonylfluorid zugegeben, worauf die Reaktionstemperatur auf 37 ° anstieg. Nach 1 h bei 24 ° hatte alles 3-Phenyl-1-propanol gemäß der Dünnschichtchromatographie reagiert. Nach Schütteln mit eiskalter NaHCO₃-Lösung, gefolgt von ges. NaCI-Lösung und ges. Citronensäurelösung und schließlich nochmals mit ges. NaCI-Lösung wurde die Toluol-Lösung mit Na₂SO₄ getrocknet und mit Hilfe von GC/MS analysiert. Dabei konnten neben wenig Allylbenzol 86 % von 3-Fluorpropylbenzol (= 1-Fluor-3-phenylpropan) nachgewiesen werden.

### 2. Herstellung von 3-α-Fluorcholestan

a) Zu einer Lösung von 0,97 g (2,5 mmol) 3-β-Hydroxycholestan und von 1,12 ml (7,5 mmol) DBU in 20 ml abs. Toluol wurden bei +2°C 1,13 g (3,75 mmol) Perfluorbutansulfonylfluorid gegeben, wobei die Temperatur auf 10 °C anstieg. Nach 1 h bei +2°C wurde abgedampft und der Rückstand in Hexan an einer Säule von 50 g Silicagel chromatographiert, wobei nach 0,23 g (25 %) Δ²(Δ³)Cholesten zuerst 0,37 g (37,75 %) reines 3-α-Fluorcholestan, Schmpt 107 - 108 °C eluiert wurde, gefolgt von 0,27 g (27,55 %) von 3-α-Fluorcholestan, das ca. 5 % 3-β-Fluorcholestan enthält. Gesamtausbeute = ca. 61 % 3-α-Fluorcholestan.
b) Bei der analogen Umsetzung in 20 ml abs. Acetonitril anstelle von 20 ml Toluol wurden nach 4 h/24 ° neben 37 % Δ²(Δ³)Cholesten und 37 % 3-α-Fluorcholestan noch 11,3 % Ausgangsmaterial (3-β-Hydroxycholestan) zurückgewonnen.
c) Bei dem analogen Versuch mit 2,0 g (7,5 mmol) Pentaisopropylguanidin anstelle von DBU in Toluol wurde 0,49 g (50 %) 3-α-Fluorcholestan erhalten.
d) Bei einem weiteren analogen Versuch mit DBN anstelle von DBU in Toluol wurden neben 41,3 % Δ²(Δ³)Cholesten ca. 41,8 % 3-α-Fluorcholestan erhalten.
e) Die gleichen Versuche mit Perfluoroktansulfonylfluorid ergaben die gleichen Ausbeuten an 3a-Fluorcholestan.

### 3. Herstellung von 3-β-Fluorandrostan

Eine Lösung von 2,38 g (5 mmol) 3-α-Hydroxyandrostan und 1,64 ml (11 mmol) DBU in 60 ml abs. Toluol wurde durch Erhitzen auf 120 ° (Ölbadtemperatur) und Abdestillieren auf 20 ml eingeengt, abgekühlt auf 24 ° und 1,66 g (5,5 mmol) Perfluorbutansulfonylfluorid zugegeben, wobei sich die Reaktionsmischung leicht erwärmte und gelb wurde. Da nach 18 h/24 ° immer noch Ausgangsmaterial vorhanden war, wurden weitere 0,3 g (2 mmol) DBU sowie 0,3 g (1 mmol) Perfluorbutansulfonylfluorid zugegeben, worauf nach 1 h/24 ° praktisch alles 3-α-Hydroxyandrostan reagiert hatte. Nach Abdampfen wurde das rohe Reaktionsprodukt in CH₂Cl₂ mit ca. 5 - 10 g Silicagel abgedampft und das Silicagel auf eine fertige mit Hexan eingeschlemmte Säule von 70 g Silicagel gegeben und mit Hexan eluiert. Die ersten 250 ml Hexan eluierten 0,57 g (44,2 %) Δ²-
(Δ³)Androsten, während die folgenden 200 ml Hexan 0,68 g (47,5 %) 3-β-Fluoran-drostan, Schmpt 93 - 95 °C und Hexan - Äther (1 : 1, 250 ml) ca. 0,070 g (5 %) nicht umgesetztes 3-α-Hydroxyandrostan ergaben.

### 4. Herstellung von 3-Methoxy-17-α-Fluor-Δ^{1,3,5(10)}-estratrien.

Zu 2,86 g (10 mmol) Estradiolmethyläther, 3,73 ml (25 mmol) DBU in 80 ml abs. Toluol wurden 3,77 g = 2,24 ml (12,5 mmol) Perfluorbutansulfonylfluorid bei 24 ° gegeben, wobei sich die Reaktionsmischung auf 32 ° erwärmte. Da nach 72 h/24 ° noch etwas Estradiolmethyläther vorhanden war, wurden nochmals 0,75 ml (5 mmol) DBU sowie 0,45 ml (2,5 mmol) Perfluorbutansulfonylfluorid zugegeben, worauf nach 2 h alles umgesetzt war. Chromatographie in Hexan an einer Säule von 50 g Silicagel ergab nach ca. 500 ml Vorlauf, der vier ungesättigte Estratriene enthielt, mit den nächsten 30 ml Hexan 0,18 g reines 3-Methoxy-17-α-fluor-Δ^{1,3,5(10)}-estratrien Schmpt. 94 - 96 ° (lit. L. H. Knox et al.; *J. Org. Chem.* **29**, 2187 (1964)) → Schmpt. 96 - 98 °, bei Umsetzung von Estradiolmethyläther mit 2-Chloro-1,1,2-trifluortriethylamin → 26 % 17-α-Fluorverbindung). GC/MS-Analyse der Mutterlaugen ergab neben den 4 möglichen ungesättigten Estratrienen insgesamt eine Ausbeute an 43 % an 3-Methoxy-17-α-fluor-Δ^{1,3,5(10)}-estratrien.

Umsetzungen von 11a-Hydroxysteroiden mit Perfluorbutansulfonylfluorid/DBU werden in Tetrahedron Letters 36, 2611 (1995) beschrieben.

## Patentansprüche

1. Verfahren zur Herstellung von Fluorverbindungen, dadurch gekennzeichnet, daß man Hydroxy-Aliphaten der allgemeinen Formel **1** mit 1 - 2 Äquivalenten Perfluorbutansulfonylfluorid **2** oder Perfluoroktansulfonylfluorid in Gegenwart von 2-3 Äquivalenten einer starken organischen (org.) Base **3** in einem indifferenten organischen Lösungsmittel zu den entsprechenden Fluorverbindungen der allgemeinen Formel **4**, in der
R₁ für einen gegebenenfalls substituierten gradkettigen oder verzweigten, aliphatischen oder araliphatischen organischen Rest und
R₂ und R₃ für Wasserstoff stehen,
oder
R₁ und R₂ gegebenenfalls substituierte gradkettige oder verzweigte aliphatische oder araliphatische Reste darstellen und
R₃ Wasserstoff ist,
oder
R₁ und R₂ einen 4 - 8 oder höhergliedrigen aliphatischen Ring bilden, der wiederum Teil eines aliphatischen oder araliphatischen polycyclischen Ringsystems sein kann und
R₃ Wasserstoff ist,
gemäß der Reaktion 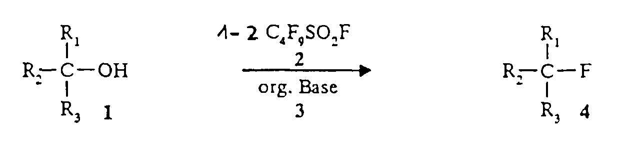 umsetzt.

2. Verfahren zur Herstellung von Fluorverbindungen gemäß Anspruch 1, dadurch gekennzeichnet, daß die aliphatischen oder araliphatischen polycyclischen Ringsysteme, zum Beispiel 3-Hydroxy- oder 17-Hydroxy- Steroide oder Hydroxyprostanester sind.

## Claims

1. Process for the preparation of fluoro compounds, characterized in that hydroxyaliphatics of the general formula **1** are reacted with 1 - 2 equivalents of perfluorobutanesulphonyl fluoride **2** or perfluorooctanesulphonyl fluoride in the presence of 2-3 equivalents of a strong organic (org.) base **3** in an indifferent organic solvent to give the corresponding fluoro compounds of the general formula **4**, in which
R₁ represents an optionally substituted straight-chain or branched, aliphatic or araliphatic organic radical and
R₂ and R₃ represent hydrogen,
or
R₁ and R₂ are optionally substituted straight-chain or branched aliphatic or araliphatic radicals and
R₃ is hydrogen,
or
R₁ and R₂ form a 4 - 8 or higher-membered aliphatic ring, which in turn can be part of an aliphatic or araliphatic polycyclic ring system and
R₃ is hydrogen,
according to the reaction

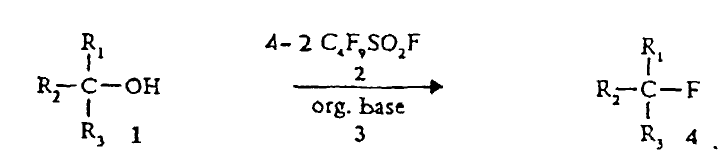

2. Process for the preparation of fluoro compounds according to Claim 1, characterized in that the aliphatic or araliphatic polycyclic ring systems are, for example, 3-hydroxy- or 17-hydroxysteroids or hydroxyprostane esters.

## Revendications

1. Procédé de préparation de composés fluorés caractérisé en ce que l'on convertit des composés hydroxyaliphatiques de formule générale 1 avec 1-2 équivalents de fluorure de perfluorobutanesulfonyle 2 ou de fluorure de perfluorooctanesulfonyle en présence de 2-3 équivalents d'une base organique forte (org.) 3 dans un solvant organique indifférent en les composés fluorés correspondants de formule générale 4 où
R₁ représente un reste organique aliphatique ou arylaliphatique linéaire ou ramifié, éventuellement substitué,
et
R₂ et R₃ représentent l'hydrogène,
ou
R₁ et R₂ représentent des restes aliphatiques ou arylaliphatiques linéaires ou ramifiés, éventuellement substitués
et
R₃ est l'hydrogène,
ou
R₁ et R₂ forment un cycle aliphatique à 4-8 chaînons ou plus, qui peut lui-même faire partie d'un système cyclique polycyclique aliphatique ou arylaliphatique
et
R₃ est l'hydrogène,
selon la réaction

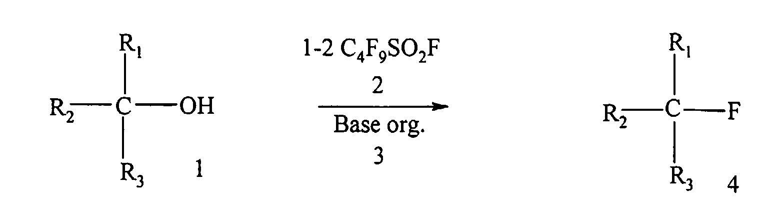

2. Procédé de préparation de composés fluorés selon la revendication 1, caractérisé en ce que les systèmes cycliques polycycliques aliphatiques ou arylaliphatiques sont par exemple des 3-hydroxy- ou 17-hydroxy-stéroïdes ou des esters d'hydroxyprostane.
